# EUROPEAN PATENT APPLICATION

(11) **EP 4 806 344 A1**
(43) Date of publication of application: **16.09.2026**
(21) Application number: 24895647.6
(22) Date of filing: 09.07.2024
(51) Int. Cl.: A61B 5/0225

(54) **PRESSURE DETECTION APPARATUS AND ELECTRONIC DEVICE**

(30) Priority: 30.11.2023 CN 202311635296
(71) Applicant: Huawei Technologies Co., Ltd., Shenzhen, Guangdong 518129 (CN)
(72) Inventor: LIU, Xin, Shenzhen, Guangdong 518129 (CN); FAN, Wei, Shenzhen, Guangdong 518129 (CN); CHEN, Mengji, Shenzhen, Guangdong 518129 (CN); ZHANG, Chuan, Shenzhen, Guangdong 518129 (CN); LIU, Yang, Shenzhen, Guangdong 518129 (CN); SHI, Jiawei, Shenzhen, Guangdong 518129 (CN)
(74) Representative: Maiwald GmbH
(86) International application number: PCT/CN2024/104468
(87) International publication number: WO 2025/112544

(57) **Abstract**

This application relates to the field of electronic device technologies, and provides a pressure detection apparatus and an electronic device. The pressure detection apparatus includes a plurality of pressure sensors; a flexible support layer, where the flexible support layer includes a first surface and a second surface that are disposed back to back along a first direction, the plurality of pressure sensors are spaced apart and attached to the first surface of the flexible support layer, and the plurality of pressure sensors are mechanically connected by using at least the flexible support layer; and one or more rigid sheets, where the one or more rigid sheets are attached to the second surface of the flexible support layer, where each of the one or more rigid sheets corresponds to a part of the pressure sensors, and a projection of each rigid sheet along the first direction covers a projection of the pressure sensor corresponding to the rigid sheet along the first direction. In this application, pressure detection precision can be improved by disposing the flexible support layer, a flexible packaging layer, and a reinforcement layer.

## Description

This application claims priority to Chinese Patent Application No. 202311635296.X, filed with the China National Intellectual Property Administration on November 30, 2023 and entitled "PRESSURE DETECTION APPARATUS AND ELECTRONIC DEVICE", which is incorporated herein by reference in its entirety.

### TECHNICAL FIELD

This application relates to the field of electronic device technologies, and in particular, to a pressure detection apparatus and an electronic device.

### BACKGROUND

Continuous long-term monitoring of blood pressure is an effective monitoring method for preventing and treating cardiovascular diseases. Currently, a blood pressure detection device is mainly a cuff-type blood pressure monitor, which obtains a blood pressure value by compressing and occluding a user's blood vessel in a cuff but can merely perform intermittent measurement on blood pressure. In addition, the large size of the cuff causes poor portability, and the squeezing force of the cuff causes discomfort to the user's arm.

With development of micro-electro mechanical system (micro-electro mechanical system, MEMS) technologies, miniaturized pressure sensors have been designed and produced. Integrating miniaturized pressure sensors into wearable devices may enable long-term and continuous detection on the user's blood pressure and effectively improve portability of a blood pressure detection apparatus.

However, current integration of miniaturized pressure sensors into wearable devices results in low accuracy of blood pressure measurement.

### SUMMARY

Some implementations of this application provide a pressure detection apparatus and an electronic device. The following describes this application from a plurality of aspects. For implementations and beneficial effects of the plurality of aspects, refer to each other.

According to a first aspect, this application provides a pressure detection apparatus. The pressure detection apparatus includes a plurality of pressure sensors; a flexible support layer, where the flexible support layer includes a first surface and a second surface that are disposed back to back along a first direction, the plurality of pressure sensors are spaced apart and attached to the first surface of the flexible support layer, and the plurality of pressure sensors are mechanically connected by using at least the flexible support layer; and one or more rigid sheets, where the one or more rigid sheets are attached to the second surface of the flexible support layer, where each of the one or more rigid sheets corresponds to a part of the plurality of pressure sensors, and a projection of each rigid sheet along the first direction covers a projection of the pressure sensor corresponding to the rigid sheet along the first direction.

According to this implementation of this application, the rigid sheet is attached to the flexible support layer. When the pressure detection apparatus is subject to pressure, the rigid sheet may restrict bending deformation of the pressure sensor, to facilitate vertical propagation of the pressure in the pressure sensor, thereby improving detection precision of the pressure detection apparatus.

In some implementations, there are a plurality of rigid sheets, and the plurality of rigid sheets are in one-to-one correspondence with the plurality of pressure sensors.

According to this implementation of this application, the rigid sheets restrict bending deformation of the pressure sensors in one-to-one correspondence, thereby further improving detection precision of the pressure detection apparatus.

In some implementations, there are a plurality of rigid sheets, and the plurality of rigid sheets are spaced apart from each other.

According to this implementation of this application, when the rigid sheets can be used to restrict bending deformation of the plurality of pressure sensors, a collision between adjacent rigid sheets is avoided when the pressure detection apparatus is bent.

In some implementations, the pressure detection apparatus further includes a flexible packaging layer, and the flexible packaging layer is disposed on a side that is of a pressure sensor layer and that faces away from the flexible support layer; and a projection of the flexible packaging layer along the first direction covers at least a projection of a part of the plurality of pressure sensors along the first direction.

According to this implementation of this application, the flexible packaging layer may be in contact with a wrist of a user. In a blood pressure detection process, a pulse wave signal can be better transmitted to the pressure sensor layer by using the flexible packaging layer. In addition, when the flexible packaging layer is attached to the wrist of the user, discomfort of the user can be reduced.

In some implementations, the flexible packaging layer includes one or more flexible sheets spaced apart, each of the one or more flexible sheets corresponds to a part of the plurality of pressure sensors, and a projection of each flexible sheet along the first direction covers a projection of the pressure sensor corresponding to the flexible sheet along the first direction.

According to this implementation of this application, the flexible sheet can better protect the pressure sensor and has higher force transmission precision.

In some implementations, there are a plurality of flexible sheets, and the plurality of flexible sheets are in one-to-one correspondence with the plurality of pressure sensors.

In some implementations, the flexible sheet is attached to the pressure sensor corresponding to the flexible sheet.

According to this implementation of this application, the flexible sheet is attached to the pressure sensor, so that the flexible sheet can better transmit a force to the pressure sensor.

In some implementations, the flexible support layer includes a flexible substrate and a conducting wire disposed on the flexible substrate, and the plurality of pressure sensors are mechanically connected by using the flexible substrate and electrically connected by using the conducting wire.

In some implementations, the plurality of pressure sensors are arranged in one or more rows, and each row includes a plurality of the pressure sensors.

According to this implementation of this application, pressure detection can be performed in different scenarios.

In some implementations, the plurality of pressure sensors are arranged in a plurality of rows, pressure sensors in a same row are spaced apart along a second direction, and the second direction is perpendicular to the first direction; and pressure sensors in two adjacent rows are staggered along a third direction, and the third direction is perpendicular to the second direction and is perpendicular to the first direction.

According to this implementation of this application, an arrangement density of the pressure sensors in a row direction can be increased, to improve detection accuracy of the pressure detection apparatus.

In some implementations, a gap between adjacent pressure sensors in a same row is 0.1 mm to 1 mm.

According to this implementation of this application, a flexible support layer located at a gap position between adjacent pressure sensors can be flexibly deformed, so that the pressure detection apparatus can be conformal with a surface of a detected object.

In some implementations, a dimension of the pressure sensor along the first direction is less than or equal to 0.5 mm; and/or a dimension of the pressure sensor along the second direction is less than or equal to 1 mm, and the second direction is perpendicular to the first direction; and/or a dimension of the pressure sensor along the third direction is less than or equal to 1 mm, and the third direction is perpendicular to the second direction and is perpendicular to the first direction.

According to this implementation of this application, while it is ensured that the arranged pressure sensors have a higher density, an overall size of the pressure detection apparatus can be reduced, and pressure detection precision can be improved.

In some implementations, a material of the flexible substrate layer includes at least one of polydimethylsiloxane, an elastomer compound, a flexible resin, and a flexible silicone.

In some implementations, the pressure sensor is an MEMS pressure sensor.

According to this implementation of this application, an array density of a sensor array can be increased, and detection precision of the pressure detection apparatus can be further improved.

In some implementations, the MEMS pressure sensor is a silicon-based piezoresistive sensor, a silicon-based piezoelectric sensor, or a silicon-based capacitive sensor.

According to a second aspect, this application further provides an electronic device. The electronic device includes a main body and the pressure detection apparatus according to the first aspect, and the pressure detection apparatus is disposed on a surface of the main body. For effects that can be achieved in the second aspect, refer to those of the pressure detection apparatus provided in any implementation of the first aspect. Details are not described herein again.

In some implementations, the electronic device is a wearable device.

In some implementations, the wearable device is a wristband-type device.

### BRIEF DESCRIPTION OF DRAWINGS

FIG. 1A is a diagram of an application scenario of a pressure detection apparatus according to an embodiment of this application;
FIG. 1B is a diagram of a cross section along A-A in FIG. 1A;
FIG. 1C is a diagram 1 of a pressure detection apparatus according to some embodiments;
FIG. 1D is a diagram 2 of a pressure detection apparatus according to some embodiments;
FIG. 2A is a three-dimensional diagram 1 of a pressure detection apparatus according to an embodiment of this application;
FIG. 2B is a sectional view along B-B in FIG. 2A;
FIG. 2C is a sectional view in which a pressure detection apparatus cooperates with a wrist according to an embodiment of this application;
FIG. 3A is a diagram 1 in which a sensor array cooperates with a blood vessel according to an embodiment of this application;
FIG. 3B is a diagram 2 in which a sensor array cooperates with a blood vessel according to an embodiment of this application;
FIG. 3C is a diagram 3 in which a sensor array cooperates with a blood vessel according to an embodiment of this application;
FIG. 4A is a top view of a pressure sensor layer according to an embodiment of this application;
FIG. 4B is a diagram in which adjacent sensors cooperate with each other according to an embodiment of this application;
FIG. 4C is a top view of FIG. 4B;
FIG. 5A is a front view of a position relationship between a flexible sheet, a rigid sheet, and a pressure sensor according to an embodiment of this application;
FIG. 5B is a projection diagram of a rigid sheet and a pressure sensor in FIG. 5A on a plane M;
FIG. 5C is a projection diagram of a flexible sheet and a pressure sensor in FIG. 5A on a plane M;
FIG. 6A is a sectional view of a structure of a pressure sensor according to an embodiment of this application;
FIG. 6B is a diagram of a deformed state of a pressure sensor under pressure when no rigid sheet is disposed according to some embodiments;
FIG. 6C is a diagram of a deformed state of a pressure sensor under pressure according to an embodiment of this application;
FIG. 7A is a three-dimensional diagram 2 of a pressure detection apparatus according to an embodiment of this application;
FIG. 7B is a sectional view along C-C in FIG. 7A;
FIG. 7C is a top view of a pressure sensor array when a pressure detection apparatus is worn on a wrist according to an embodiment of this application;
FIG. 7D is a top view of a pressure sensor array when a pressure detection apparatus is worn on a wrist according to some embodiments;
FIG. 8A is a sectional view 1 of a pressure detection apparatus according to an embodiment of this application;
FIG. 8B is a sectional view 2 of a pressure detection apparatus according to an embodiment of this application;
FIG. 9A is a sectional view 3 of a pressure detection apparatus according to an embodiment of this application;
FIG. 9B is a sectional view 4 of a pressure detection apparatus according to an embodiment of this application; and
FIG. 9C is a sectional view 5 of a pressure detection apparatus according to an embodiment of this application.

### DESCRIPTION OF EMBODIMENTS

The following describes specific implementations of this application in detail with reference to accompanying drawings.

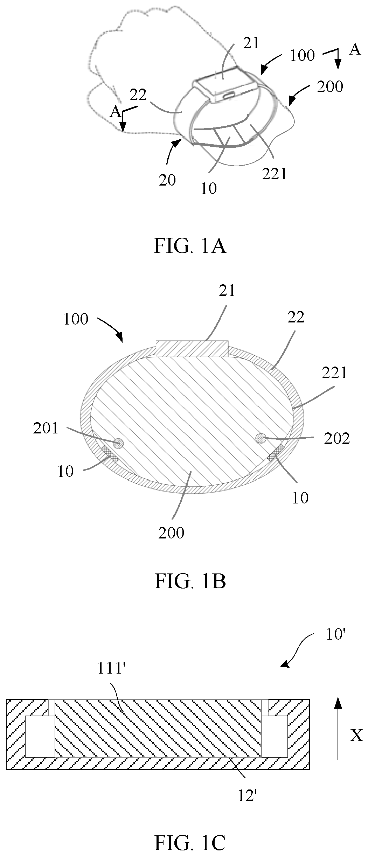

An embodiment of this application is used to provide a pressure detection apparatus, to improve detection precision. FIG. 1A and FIG. 1B show example application scenarios of a pressure detection apparatus according to an embodiment of this application. The pressure detection apparatus is specifically a wearable device 100 configured to detect blood pressure. The wearable device 100 may be a wristband-type device (for example, a smartwatch or a smart band), an armband-type device (for example, a device worn on an upper arm or a lower arm), a head-mounted device, a finger wearable device, an intelligent pressure sensing patch (for example, a patch that may be attached to a brachial artery or a carotid artery), or the like. This is not specifically limited in this application. The following provides detailed descriptions by using an example in which the wearable device 100 is a wristband-type device.

It should be noted that FIG. 1A and FIG. 1B show merely example application scenarios of this embodiment of this application. In another example, a pressure detection apparatus 10 may be used in another device. For example, the pressure detection apparatus 10 may be disposed on a surface of a terminal device such as a headset or a stylus. When a user uses the terminal device, the pressure detection apparatus 10 may detect user pressure (for example, holding pressure of the user on the stylus, or contact pressure between the headset and an ear of the user). For another example, the pressure detection apparatus 10 may be used on a surface of a steering wheel or a joystick of a smart cockpit, and is configured to detect user operation pressure, to help the smart cockpit perform haptic feedback and sensing recognition on a user operation. For another example, the pressure detection apparatus 10 may be disposed on a surface of a robot, serves as a haptic sensor of the robot, and is configured for haptic feedback recognition of the robot. For example, the pressure detection apparatus 10 may be further disposed on a surface of a pipeline, and is configured to detect impact pressure of fluid (for example, seawater) in the pipeline on a pipeline wall, to help perform detection and fault location on the pipeline. In addition, the pressure detection apparatus 10 may be further applied to other fields such as the industrial field, the aviation field, and the aerospace field. Details are not described one by one.

FIG. 1A and FIG. 1B show an example structure of a wearable device 100 according to an embodiment of this application. FIG. 1A is a diagram of a three-dimensional structure of a wearable device 100. FIG. 1B is a diagram of a cross section along A-A in FIG. 1A. In addition, for ease of understanding, transparency processing is performed on a wrist 200 of the user in FIG. 1A, to help observe a disposing position of the pressure detection apparatus 10.

As shown in FIG. 1A and FIG. 1B, the wearable device 100 provided in this embodiment of this application may include a main body 20 and the pressure detection apparatus 10. The main body 20 may include a watch body 21 and a wristband 22. The watch body 21 is configured to implement a main function of the wearable device 100, for example, display, audio playing, and communication.

The wristband 22 is configured to wear the watch body 21 on the wrist 200 of the user. The wristband 22 has a deformation capability, and may be adaptively bent based on a shape of the wrist 200. For example, the wristband 22 may be made of a flexible material such as knitted fabric, leather, or plastic, or may be obtained by combining a plurality of metal blocks that are rotatably connected, so that the wristband 22 can extend around the wrist 200 of the user, to wear the watch body 21 on the wrist 200 of the user. The watch body 21 and the wristband 22 may be connected in various manners such as a snap-fitting manner, a bonding manner, and a clamping manner, and may be detachably connected or fastened. This is not limited in this application.

The pressure detection apparatus 10 may be disposed on an inner surface 221 of the wristband 22. The inner surface 221 of the wristband 22 is a surface that is of the wristband 22 and that faces the user when the wearable device 100 is in a worn state. When the user wears the wearable device 10, the pressure detection apparatus 10 may be aligned with a blood vessel of the wrist 200 of the user, to detect blood pressure of the user.

In this embodiment of this application, one pressure detection apparatus 10 or a plurality of pressure detection apparatuses 10 may be disposed on the wearable device 100. As shown in FIG. 1B, two pressure detection apparatuses 10 are disposed on the wearable device 100. The two pressure detection apparatuses 10 may be respectively aligned with a wrist blood vessel 201 and a wrist blood vessel 202, to respectively detect blood pressure in the blood vessel 201 and blood pressure in the blood vessel 202. Blood pressure detection results of the plurality of pressure detection apparatuses 10 may be mutually verified, thereby improving accuracy of blood pressure measurement.

FIG. 1C shows an example structure of a pressure detection apparatus 10' in some embodiments. As shown in FIG. 1C, the pressure detection apparatus 10' includes a pressure sensor 111' and a rigid packaging structure 12'. In a blood pressure detection process, a surface of the pressure sensor 111' may be press-fitted on a surface of a user body, to obtain blood pressure of the user through measurement based on an applanation tonometry method. In this embodiment, there is one pressure sensor 111' in the pressure detection apparatus 10'. Therefore, the pressure sensor 111' needs to be precisely aligned with a user blood vessel to obtain blood pressure of the user through measurement. In addition, the pressure sensor 111' is rigidly packaged. In a blood pressure measurement process, mechanical pressure needs to be applied to the pressure sensor 111', and the mechanical pressure causes discomfort to the user.

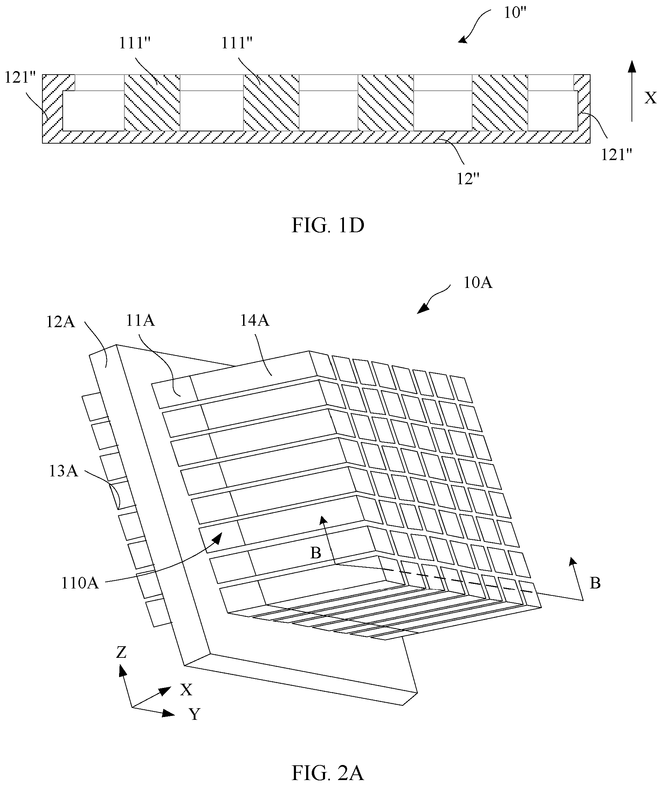

FIG. 1D shows an example structure of a pressure detection apparatus 10" in some other embodiments. As shown in FIG. 1D, the pressure detection apparatus 10" includes a sensor array including a plurality of pressure sensors 111" and a rigid packaging structure 12". In this embodiment, the pressure detection apparatus 10" may measure blood pressure of the user based on an oscillometric method or an applanation tonometry method. However, a shear wall 121" of the rigid packaging structure 12" limits pressure conduction of a part of the pressure sensors 111", affects blood pressure detection precision of the pressure detection apparatus 10", and brings a strong sense of pressure to the user. In addition, the rigid packaging structure 12" limits an array density of the pressure sensors 111". For example, a spacing between adjacent pressure sensors 111" can only be at a millimeter level. Further reducing the array density greatly increases processing difficulty and processing costs, and a low-density sensor array limits blood pressure detection precision of the pressure detection apparatus 10".

Therefore, an embodiment provides a pressure detection apparatus, including a pressure sensor layer, a flexible support layer, and a reinforcement layer that are sequentially stacked. The pressure sensor layer includes a pressure sensor array including a plurality of pressure sensors. The flexible support layer may provide flexible support for the pressure sensor array. In a pressure (for example, blood pressure) detection process, the flexible support layer may generate corresponding deformation based on a shape of a surface of a detected object (for example, a user body), to implement conformality between the pressure detection apparatus and the detected object. Compared with the rigid packaging structures in FIG. 1C and FIG. 1D, in this embodiment, accuracy of pressure detection can be improved, and a sense of pressure of the user can be reduced.

In addition, the pressure detection apparatus further includes the reinforcement layer, the reinforcement layer may include one or more rigid sheets, and the rigid sheet is aligned with the pressure sensor along a stacking direction. When the pressure detection apparatus is bent, the rigid sheet may restrict transverse deformation of the pressure sensor, to implement vertical propagation of pressure in the pressure sensor, and further improve detection precision of the pressure detection apparatus.

In some embodiments, the pressure sensor is an MEMS sensor. Because the MEMS sensor may be in an extremely small size, for example, a size below a millimeter level, to increase an array density of the sensor array, and further improve detection precision of the pressure detection apparatus.

The following describes Embodiment 1 of the pressure detection apparatus provided in this application.

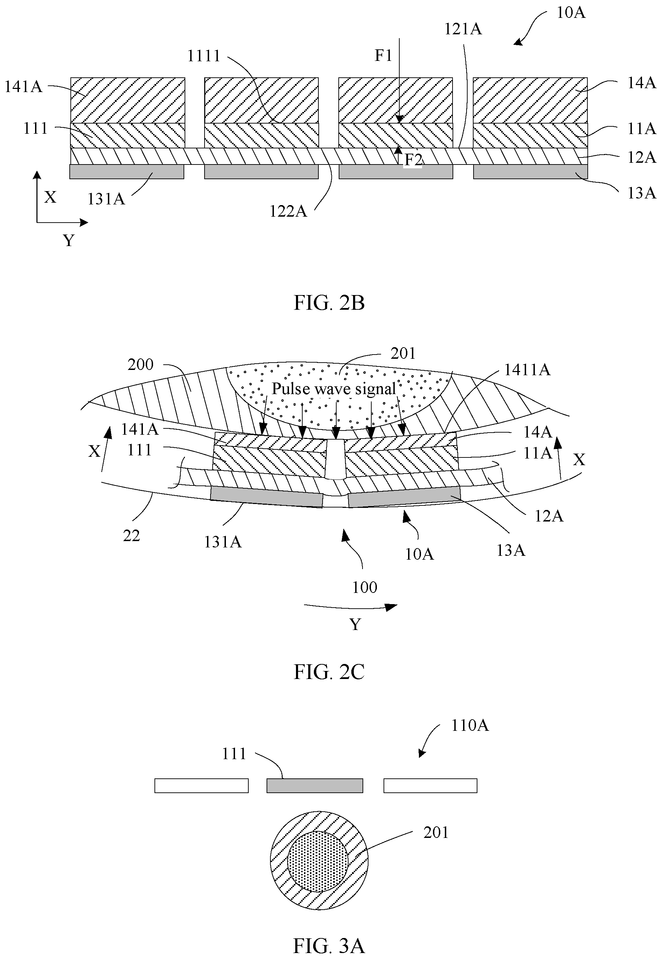

FIG. 2A to FIG. 2C are diagrams of an example structure of a pressure detection apparatus 10A according to Embodiment 1 of this application. FIG. 2A is a three-dimensional diagram of a pressure detection apparatus 10A. FIG. 2B is a sectional view along B-B in FIG. 2A. FIG. 2C shows a position relationship between a pressure detection apparatus 10A and a wrist 200 of a user when a wearable device 100 is worn on the wrist 200 of the user.

In figures in this specification, an X-axis direction represents a thickness direction of the pressure detection apparatus 10A or a stacking direction (which is used as a first direction) of layers of structures of the pressure detection apparatus 10A, a Y-axis direction is a length direction (which is used as a second direction) of the pressure detection apparatus 10A, and a Z-axis direction is a width direction (which is used as a third direction) of the pressure detection apparatus 10A, which are not independently emphasized subsequently. The X-axis direction, the Y-axis direction, and the Z-axis direction may be perpendicular to each other.

In addition, for ease of understanding, in this specification, a surface of a side of each structure facing a positive direction of an X-axis is referred to as a front surface of the structure, and a surface of a side of each structure facing away from the positive direction of the X-axis is referred to as a back surface of the structure. A length, a width, and a thickness of each part are respectively dimensions of the part along the Y-axis direction, the Z-axis direction, and the X-axis direction.

In addition, in the following descriptions, a blood vessel 201 of the wrist 200 is used as an example of a to-be-detected blood vessel. It may be understood that, this application is not limited thereto. In another example, the to-be-detected blood vessel may be another blood vessel of a user body.

As shown in FIG. 2A and FIG. 2B, the pressure detection apparatus 10A includes a flexible packaging layer 14A, a pressure sensor layer 11A, a flexible support layer 12A, and a reinforcement layer 13A that are sequentially stacked along the thickness direction. As shown in FIG. 2C, when the wearable device 100 is worn on the wrist 200 of the user, the thickness direction (namely, the X-axis direction) of the pressure detection apparatus 10A may be perpendicular or basically perpendicular to a surface of the wrist 200 of the user, and the length direction (namely, the Y-axis direction) of the pressure detection apparatus 10A may be parallel or basically parallel to a circumference direction of the wrist 200 of the user.

In addition, when the pressure detection apparatus 10A is disposed on the wearable device 100, the pressure detection apparatus 10A is located between the wrist 200 of the user and the wristband 22 of the wearable device 100. The flexible packaging layer 14A is located on a side that is of the pressure detection apparatus 10A and that faces the wrist 200 of the user, and the reinforcement layer 13A is located on a side that is of the pressure detection apparatus 10A and that faces the wristband 22 of the wearable device 100. For example, the reinforcement layer 13A may be attached to an inner surface 221 of the wristband 22, and the flexible packaging layer 14A may be configured to be in contact (for example, attached) with the wrist 200 of the user. In a blood pressure detection process, a pulse wave signal in the blood vessel of the wrist 200 may be transmitted to the pressure sensor layer 11A by using the flexible packaging layer 14A.

The following specifically describes layers of structures of each pressure detection apparatus 10A with reference to FIG. 2A to FIG. 2C.

An example structure of the pressure sensor layer 11A is first described. As shown in FIG. 2A and FIG. 2B, the pressure sensor layer 11A may include a plurality of pressure sensors 111. Each pressure sensor 111 may sense pressure acting on a measurement surface 1111 (namely, an upper surface of the pressure sensor 111) of the pressure sensor 111, and output a corresponding electrical signal based on a magnitude of the sensed pressure. For example, the pressure sensor 111 may output a current signal or a voltage signal in direct proportion to the magnitude of the pressure. Because the pressure sensor layer 11A includes the plurality of pressure sensors 111, and it may be understood that the pressure detection apparatus 10A may detect and obtain the pulse wave signal in the blood vessel 201 when at least one pressure sensor 111 in the pressure sensor layer 11A is aligned with the wrist blood vessel, compared with a single pressure sensor solution in FIG. 1C, in this embodiment of this application, the provided pressure detection apparatus 10A can reduce a requirement on alignment precision between the pressure detection apparatus 10A and the blood vessel 201.

Further, as shown in FIG. 2A and FIG. 2B, the plurality of pressure sensors 111 may be arranged in an array. In other words, the plurality of pressure sensors 111 may be arranged in a plurality of rows and a plurality of columns, thereby forming a sensor array 110A. The Y-axis direction is a row direction of the sensor array 110A, and the Z-axis direction is a column direction of the sensor array 110A. As shown in FIG. 2A and FIG. 2B, each row of the sensor array 110A may include a plurality of pressure sensors 111.

In another example, the sensor array 110A may alternatively have one row and a plurality of columns, one row includes a plurality of pressure sensors 111, and each pressure sensor 111 is used as one column. It may be understood that a row quantity and a column quantity of the sensor array 110A may be adaptively adjusted according to a requirement, for example, one row and six columns, three rows and nine columns, or eight rows and eight columns, provided that a same row includes a plurality of pressure sensors 111. This is not specifically limited.

It may be understood that when the pressure detection apparatus 10A is disposed on the wearable device 100, the row direction (namely, the X-axis direction) of the sensor array 110A may be parallel or basically parallel to an extension direction of the wristband 22 of the wearable device 100. In this way, when the wearable device 100 is worn on the wrist 200 of the user, the row direction of the sensor array 110A may be parallel or basically parallel to the circumference direction of the wrist 200 of the user.

Usually, an extension direction of the blood vessel 201 is approximately parallel to an extension direction of an arm, in other words, approximately perpendicular to the circumference direction of the wrist 200. Therefore, in this embodiment, a plurality of pressure sensors 111 are disposed in the row direction of the sensor array 110A. In this way, when the wearable device 100 is worn on the wrist 200 of the user, it is easier to ensure that the requirement on alignment precision between the pressure detection apparatus 10A and the blood vessel 201 is further reduced when at least one of pressure sensors 111 in a same row is aligned with the blood vessel 201 of the wrist 200.

FIG. 3A to FIG. 3C show impact on a blood pressure measurement result when the sensor array 110A is set to different densities. From FIG. 3A to FIG. 3C, array densities of the sensor array 110A increase sequentially. In addition, for ease of observation, in FIG. 3A to FIG. 3C, a pressure sensor 111 aligned with the blood vessel 201 is shown by using a rectangle with a shadow, and a pressure sensor 111 not aligned with the blood vessel 201 is shown by using a rectangle without a shadow.

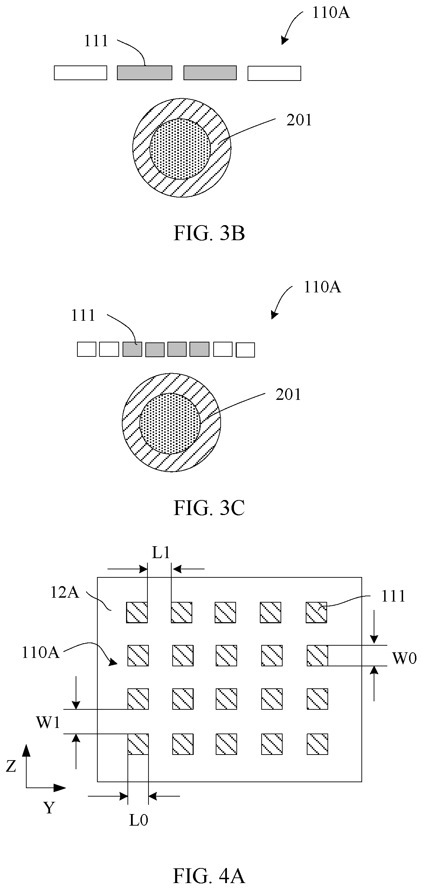

As shown in FIG. 3A, when the sensor array 110A has an array density ρ1, one pressure sensor 111 in a same row may be aligned with the blood vessel 201. As shown in FIG. 3B, when the sensor array 110A has an array density ρ2 (ρ2>ρ1), two pressure sensors 111 in a same row may be aligned with the blood vessel 201. As shown in FIG. 3C, when the sensor array 110A has an array density ρ3 (ρ3>ρ2), four pressure sensors 111 in a same row may be aligned with the blood vessel 201. That is, a higher array density of the sensor array 110A better helps reduce the requirement on alignment precision between the pressure detection apparatus 10A and the blood vessel 201.

Therefore, in some embodiments of this application, an MEMS sensor that may be in an extremely small size is used as a pressure sensor 111, to increase the array density of the sensor array 110A as much as possible. The MEMS pressure sensor may be a silicon-based piezoresistive sensor, a silicon-based piezoelectric sensor, a silicon-based capacitive sensor, or the like. This is not specifically limited.

FIG. 4A is a top view of the pressure sensor layer 11A, and shows an example size setting manner of a pressure sensor array 110A according to an embodiment of this application. As shown in FIG. 4A, a length L0 of a single pressure sensor 111 may not exceed 1 mm (for example, 0.8 mm or 1 mm), a width WO may not exceed 1 mm (for example, 0.8 mm or 1 mm), and a thickness may not exceed 0.5 mm (for example, 0.4 mm or 0.5 mm).

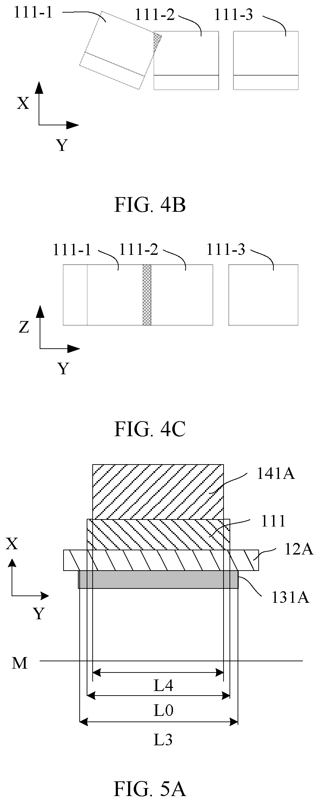

In addition, because the pressure sensor 111 has a specific thickness, when the wearable device 10A is worn on the wrist 200 of the user, interference may occur between adjacent pressure sensors 111 in a same row. For example, as shown in FIG. 4B and FIG. 4C (FIG. 4C is a top view of FIG. 4B), when the wearable device 10A is worn on the wrist 200 of the user, interference occurs between a pressure sensor 111-1 and a pressure sensor 111-2 that are adjacent to each other in a same row. For ease of observation, FIG. 4B and FIG. 4C show interference regions between adjacent sensors 111 by using shadow parts.

Therefore, in this embodiment of this application, there may be a gap between adjacent pressure sensors 111 in a same row. For example, as shown in FIG. 4A, a gap L1 (which is used as a first gap) between adjacent pressure sensors 111 in a same row may be 0.1 mm to 1 mm (for example, 0.3 mm or 1 mm). A gap W1 (which is used as a first gap) between adjacent pressure sensors 111 in a same column may not exceed 0.1 mm to 1 mm (for example, 0.3 mm or 1 mm).

In this embodiment of this application, the array density of the sensor array 110A is increased, to reduce the requirement on alignment precision between the pressure detection apparatus 10A and the blood vessel 201. In addition, because the sensor array 110A has a high array density, more pressure sensors 111 in the sensor array 110A are aligned with the blood vessel 201, thereby improving blood pressure measurement precision of the pressure detection apparatus 10A.

The following describes an example structure of the flexible support layer 12A. As shown in FIG. 2A and FIG. 2B, the flexible support layer 12A includes a surface 121A (which may also be referred to as a front surface 121A of the flexible support layer 12A, and is used as a first surface) and a surface 122A (which may also be referred to as a back surface 122A of the flexible support layer 12A, and is used as a second surface) that are disposed back to back along the thickness direction. The pressure sensor layer 11A is disposed on the surface 121A, and the reinforcement layer 13A is disposed on the surface 122A.

The flexible support layer 12A is configured to provide flexible support for the pressure sensor layer 11A. For example, as shown in FIG. 2B, when the pressure sensor 111 is subject to downward pressure F1, the flexible support layer 12A may provide an upward acting force F2 for the pressure sensor 111. In addition, the flexible support layer 12A has a deformation capability. As shown in FIG. 2C, when the wearable device 100 is worn on the wrist 200 of the user, the flexible support layer 12A can generate deformation that adapts to a shape of the wrist 200 of the user, to implement conformality between the pressure detection apparatus 10A and the wrist 200 of the user, so that each pressure sensor 111 can be attached to the surface of the wrist 200 of the user. In this way, the measurement surface 1111 (namely, a front surface of the pressure sensor 111) of the pressure sensor 111 may be basically perpendicular to a normal direction of the surface of the wrist 200 of the user, so that the pulse wave signal in the blood vessel 201 is vertically transmitted to the pressure sensor 111 (that is, transmitted to the pressure sensor 111 along a normal direction of the measurement surface 1111), thereby improving detection accuracy of the pressure detection apparatus 10A.

The flexible support layer 12A may be prepared by using a flexible material having a flexible deformation capability, for example, polyimide (Polyimide, PI), polyethylene terephthalate (PET), polydimethylsiloxane (Polydimethylsiloxane, PDMS), flexible resin, or flexible silicone. This is not specifically limited.

In some embodiments, the flexible support layer 12A is at least configured to provide a mechanical connection for the plurality of pressure sensors 111 in the pressure sensor layer 11A. For example, each pressure sensor 111 may be mechanically connected to the flexible support layer 12A. In this way, the plurality of pressure sensors 111 in the pressure sensor layer 11A may be mechanically (or referred to as "physically") interconnected by using the flexible support layer 12A, so that the plurality of pressure sensors 111 are not physically separated from each other. A mechanical connection manner between the pressure sensors 111 and the flexible support layer 12A may include bonding, welding, snap-fitting, or the like. This is not limited in this application.

In some other embodiments, the flexible support layer 12A is not only configured to provide a mechanical connection for the plurality of pressure sensors 111 in the pressure sensor layer 11A, but also can provide an electrical connection for the plurality of pressure sensors 111. For example, the flexible support layer 12A includes a flexible substrate and a conducting wire. The flexible substrate may be prepared by using the foregoing flexible material (for example, PI or PET) that has a deformation capability, and the conducting wire may be formed on the flexible substrate by using a printed circuit. The plurality of pressure sensors 111 in the pressure sensor layer 11A may be mechanically connected by using the flexible substrate, and electrically connected by using the conducting wire. For example, an electrode of the pressure sensor 111 may be electrically connected to the conducting wire in the flexible support layer 12A through gold wire bonding, or an electrode of the pressure sensor 111 may be welded to the conducting wire in the flexible support layer 12A by using a patch, to implement an electrical connection between the electrode of the pressure sensor 111 and the conducting wire in the flexible support layer 12A.

In some embodiments, a thickness of the flexible support layer 12A may be less than or equal to 0.5 mm, to ensure that the flexible support layer 12A has a strong flexible deformation capability while meeting a support capability for the pressure sensor layer 11A.

In this embodiment of this application, the flexible support layer 12A may be disposed to implement a connection of the sensor array 110A, and improve relative stability of the plurality of pressure sensors 111. In addition, when the wearable device 100 is worn on the wrist 200, the flexible support layer 12A can generate deformation with a shape of the wrist 200, so that the measurement surface 1111 of the pressure sensor 111 can be basically perpendicular to a transmission direction of the pulse wave signal. In this way, the pulse wave signal in the blood vessel 201 is vertically transmitted to the pressure sensor 111, thereby improving detection accuracy of the pressure detection apparatus 10A.

The following describes an example structure of the reinforcement layer 13A. As shown in FIG. 2A and FIG. 2B, the reinforcement layer 13A may include a plurality of rigid sheets 131A, and each rigid sheet 131A is attached to a back surface 122A of the flexible support layer 11, for example, is attached to the back surface 122A of the flexible support layer 12A through bonding, welding, or the like. A back surface of the rigid sheet 131A may be configured to be connected to the wristband 22.

For example, as shown in FIG. 2A and FIG. 2B, a quantity of rigid sheets 131A may be the same as a quantity of pressure sensors 111. The plurality of rigid sheets 131A are in one-to-one correspondence with the plurality of pressure sensors 111. A projection of each rigid sheet 131A along the thickness direction covers a projection of a pressure sensor 111 corresponding to the rigid sheet 131A along the thickness direction.

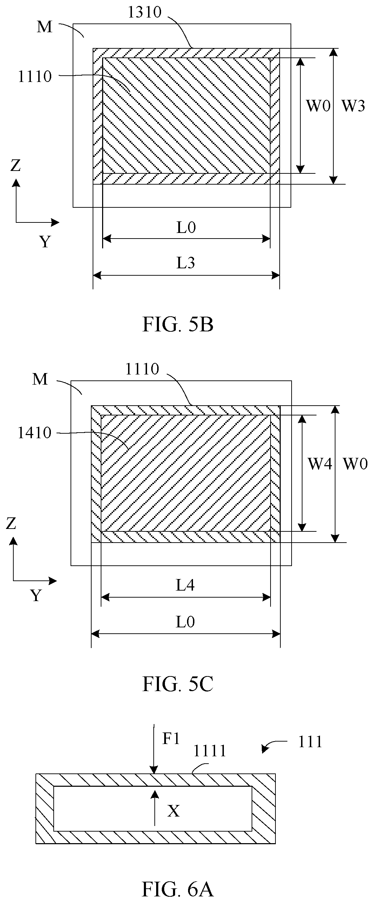

For ease of understanding, FIG. 5A and FIG. 5B are diagrams of a position relationship between a corresponding rigid sheet 131A and a corresponding pressure sensor 111. FIG. 5A is a front view of the rigid sheet 131A and the pressure sensor 111. FIG. 5B is a projection diagram of the rigid sheet 131A and the pressure sensor 111 on a plane M. The plane M is perpendicular to the thickness direction of the pressure detection apparatus 10A.

As shown in FIG. 5A and FIG. 5B, a projection of the pressure sensor 111 on the plane M along the thickness direction is a projection 1110, and a projection of the rigid sheet 131 on the plane M along the thickness direction is a projection 1310. The projection 1110 of the pressure sensor 111 has a length L0, and the projection 1310 of the rigid sheet 131A has a length L3, where L3>L0. The projection 1110 of the pressure sensor 111 has a width WO, and the projection 1310 of the rigid sheet 131A has a width W3, where W3>WO. A shadow area of the projection 1110 of the pressure sensor 111 is less than a shadow area of the projection 1310 of the rigid sheet 131A, and the shadow area of the projection 1110 is located in the shadow area of the projection 1310. In other words, the projection of the rigid sheet 131A along the thickness direction covers the projection of the pressure sensor 111 corresponding to the rigid sheet 131A along the thickness direction.

In some embodiments, a length and a width of the rigid sheet 131A may be respectively the same as a length and a width of the corresponding pressure sensor 111. In some other embodiments, a length of the rigid sheet 131A may be slightly greater than a length of the corresponding pressure sensor 111, and/or a width of the rigid sheet 131A may be slightly greater than a width of the corresponding pressure sensor 111.

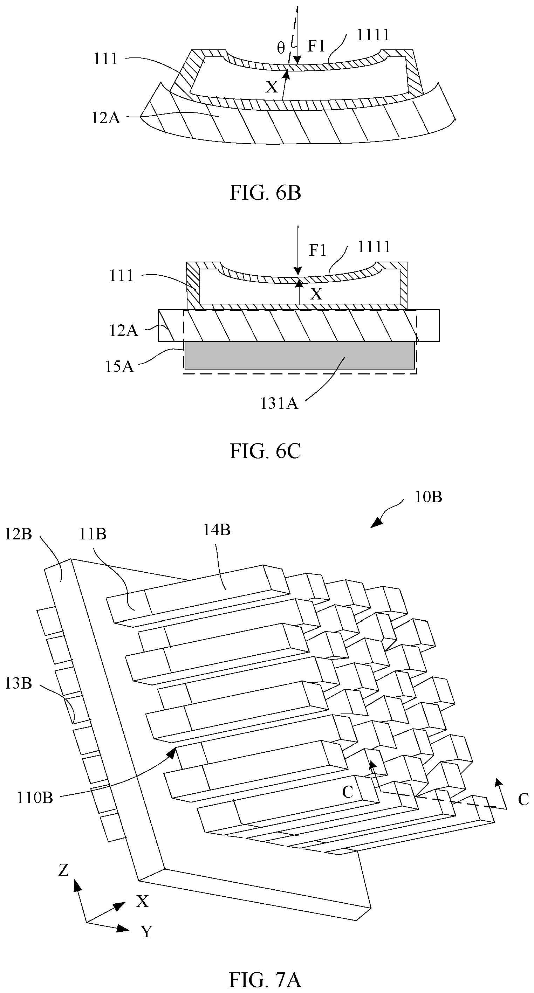

The following describes an example of a function of the rigid sheet 131A with reference to FIG. 6A to FIG. 6C. FIG. 6A shows an example structure of the pressure sensor 111A. FIG. 6B shows a deformed state of a pressure sensor 111A under pressure when no rigid sheet 131A is disposed. FIG. 6C is a diagram of a deformed state of a pressure sensor 111A under pressure when a rigid sheet 131A is disposed.

As shown in FIG. 6A and FIG. 6B, the pressure sensor 111 is an elastic structure having a specific thickness. When the measurement surface 1111 of the pressure sensor 111 is subject to the downward pressure F1, the pressure sensor 111 generates bending deformation, that is, each part of the pressure sensor 111 generates transverse deformation (deformation perpendicular to an acting direction of the pressure F1) to different degrees. For example, an upper partial structure of the pressure sensor 111 generates transverse compression deformation, and a lower partial structure generates transverse tensile deformation. After the pressure sensor 111 generates bending deformation, a direction of the pressure F1 may be no longer parallel to the thickness direction of the pressure sensor 111, but an included angle θ is formed between the direction of the pressure F1 and the thickness direction of the pressure sensor 111. In other words, the pressure F1 cannot be vertically transmitted in the pressure sensor 111, thereby affecting detection precision of the pressure sensor 111.

Therefore, in this embodiment of this application, the pressure detection apparatus 10A further includes the rigid sheet 131A disposed on the back surface of the flexible support layer 12A. The rigid sheet 131A has large rigidity. For example, the rigidity of the rigid sheet 131A may be represented by an elastic modulus of the rigid sheet 131A. In some embodiments, the elastic modulus of the rigid sheet 131A may be far greater than an elastic modulus of the flexible support layer 12A. For example, the elastic modulus of the rigid sheet 131A is more than five times the elastic modulus of the flexible support layer 12A. In some other embodiments, the elastic modulus of the rigid sheet 131A may be greater than a specified value, for example, greater than 50 GPa. For example, a material of the rigid sheet 131A may be a metal material (for example, steel or aluminum), rigid plastic, glass, or the like. This is not limited in this application.

As shown in FIG. 6C, after the rigid sheet 131A is attached to the back surface 122A of the flexible support layer 12A, a part that is of the flexible support layer 12A and that is attached to the rigid sheet 131A and the rigid sheet 131A jointly form a constraint structure 15A for the pressure sensor 111. It may be understood that rigidity of the constraint structure 15A is basically the same as the rigidity of the rigid sheet 131A. In this way, when the pressure sensor 111 is subject to the pressure F1, the lower partial structure of the pressure sensor 111 is constrained by the constraint structure 15A. To be specific, under an action of the pressure F1, the lower partial structure of the pressure sensor 111 almost does not generate transverse deformation. Therefore, the pressure sensor 111 almost does not generate bending deformation as a whole. In this way, the pressure F1 may be propagated along the thickness direction of the pressure sensor 111. In other words, vertical propagation of the pressure F1 in the pressure sensor 111 is implemented, to improve detection precision of the pressure sensor 111.

Further, as shown in FIG. 2A to FIG. 2C, the plurality of rigid sheets 131A may be spaced apart. In other words, there may be a gap between adjacent rigid sheets 131A. In this way, after the wearable device 100 is worn on the wrist 200 of the user, a part that is of the flexible support layer 12A and that corresponds to the gap may be flexibly deformed, so that the pressure detection apparatus 10A may be conformal with the wrist 200 of the user.

In some embodiments, a thickness of the rigid sheet 131A may not exceed 1 mm, to reduce an overall thickness of the pressure detection apparatus 10A. For example, the thickness of the rigid sheet 131A is 0.8 mm or 1 mm.

In this embodiment of this application, the reinforcement layer 13A disposed on the back surface 122A of the flexible support layer 12A can provide a transverse constraint force when the pressure sensor 111 is subject to pressure, to improve structural stability of the pressure sensor 111, implement vertical propagation of pressure in the pressure sensor 111, and improve blood pressure detection precision.

The following describes an example structure of the flexible packaging layer 14A. As shown in FIG. 2A to FIG. 2C, the flexible packaging layer 14A is disposed on a side of a front surface of the pressure sensor layer 11A, and a projection of the flexible packaging layer 14A along the thickness direction covers a projection of the pressure sensor 111 along the thickness direction.

The flexible packaging layer 14A has a flexible deformation capability. As shown in FIG. 2C, when the wearable device 100 is worn on the wrist 200 of the user, the flexible packaging layer 14A can generate deformation adapted to the shape of the wrist 200 of the user, so that a front surface of the flexible packaging layer 14A is conformal with the surface of the wrist 200 of the user. In this way, the measurement surface 1111 of each pressure sensor 111 may be basically perpendicular to the normal direction of the surface of the wrist 200 of the user, which facilitates vertical transmission of the pulse wave signal in the blood vessel to the pressure sensor 111, thereby improving detection accuracy of the pressure detection apparatus.

The flexible packaging layer 14A may be prepared by using a flexible material having a flexible deformation capability, for example, PDMS, flexible resin, flexible silicone, or biodegradable plastic. This is not specifically limited.

In some embodiments, a flexible deformation capability of the flexible packaging layer 14A may be greater than or equal to a flexible deformation capability of the flexible support layer 12A, to achieve better conformal effect between the front surface of the flexible packaging layer 14A and the surface of the wrist 200 of the user, reduce discomfort of the wrist 200 of the user, and improve user experience. In addition, the flexible packaging layer 12A may further protect the pressure sensor layer 11A.

In some embodiments, the flexible packaging layer 14A may include a plurality of flexible sheets 141A, and each flexible sheet 141A is attached to the front surface of the pressure sensor 111, for example, may be attached to the front surface of the pressure sensor 111 through packaging, bonding, or the like. A front surface of the flexible sheet 141A (namely, a packaging surface 1411A of the flexible sheet 141A) is configured to be attached to the surface of the wrist 200, and the front surface of the flexible sheet 141A can be conformal with the surface of the wrist 200. In another embodiment, another structure may also be disposed between the flexible sheet 141A and the pressure sensor 111.

For example, as shown in FIG. 2A and FIG. 2B, a quantity of flexible sheets 141A may be the same as a quantity of pressure sensors 111. The plurality of flexible sheets 141A are in one-to-one correspondence with the plurality of pressure sensors 111. A projection of each flexible sheet 141A along the thickness direction covers a projection of a pressure sensor 111 corresponding to the flexible sheet 141A along the thickness direction.

For ease of understanding, FIG. 5A and FIG. 5C are diagrams of a position relationship between a corresponding flexible sheet 141A and a corresponding pressure sensor 111. FIG. 5A is a front view of the flexible sheet 141A and the pressure sensor 111. FIG. 5C is a projection diagram of the flexible sheet 141A and the pressure sensor 111 on a plane M. The plane M is perpendicular to the thickness direction of the pressure detection apparatus 10A.

To be specific, as shown in FIG. 5A and FIG. 5C, a projection of the flexible sheet 141A on the plane M along the thickness direction is a projection 1410, and a projection of the pressure sensor 111 on the plane M along the thickness direction is a projection 1110. The projection 1110 of the pressure sensor 111 has a length L0, and the projection 1410 of the flexible sheet 141A has a length L4, where L0>L4. The projection 1110 of the pressure sensor 111 has a width WO, and the projection 1410 of the flexible sheet 141A has a width W4, where WO>W4. A shadow area of the projection 1110 of the pressure sensor 111 is greater than a shadow area of the projection 1410 of the flexible sheet 141A, and the shadow area of the projection 1410 is located in the shadow area of the projection 1110. In other words, the projection of the pressure sensor 111 along the thickness direction covers the projection of the flexible sheet 141A corresponding to the pressure sensor 111 along the thickness direction.

In some embodiments, a length L4 and a width W4 of the flexible sheet 141A may be respectively the same as a length L0 and a width WO of the corresponding pressure sensor 111. In some other embodiments, a length L4 of the flexible sheet 141A may be slightly greater than a length L0 of the corresponding pressure sensor 111, and/or a width W4 of the flexible sheet 141A may be slightly greater than a width WO of the corresponding pressure sensor 111. In this way, the projection of the flexible sheet 141A along the thickness direction covers the projection of the corresponding pressure sensor 111 along the thickness direction, so that the flexible sheet 141A can transmit a force while better protecting the pressure sensor 111.

The front surface of the flexible sheet 141A is configured to be conformal with the surface of the wrist 200. For example, as shown in FIG. 2C, when the wearable device 100 is worn on the wrist 200 of the user, a packaging surface 1411A of the flexible sheet 141A is in contact with the surface of the wrist 200, and can generate corresponding deformation with the shape of the surface of the wrist 200. For example, the surface of the wrist 200 has a protrusion. When the packaging surface 141A is attached to the surface of the wrist 200, a concave matching the protrusion is formed on the packaging surface 141A, so that the packaging surface 141A is approximately or completely attached to the surface of the wrist 200. In this case, a thickness direction of the flexible sheet 141A is consistent with the thickness direction of the pressure sensor 111. When the pulse wave signal in the blood vessel 201 is transmitted to the pressure sensor 111 by using the flexible sheet 141A, pressure on the measurement surface 1111 of the pressure sensor 111 may be basically perpendicular to the normal direction of the measurement surface 1111, to facilitate vertical propagation of the pressure in the pressure sensor 1111 and improve measurement precision of the pressure sensor 111.

Further, as shown in FIG. 2A to FIG. 2C, the plurality of flexible sheets 141A may be spaced apart. In other words, there may be a gap between adjacent flexible sheets 141A. In this way, after the wearable device 100 is worn on the wrist 200 of the user, the gap between adjacent flexible sheets 141A enables the packaging surface 1411A of the flexible sheet 141A to be better adapted to the shape of the wrist surface, so that the pressure detection apparatus 10A can be conformal with the wrist 200 of the user.

Further, it is considered that if a thickness of the flexible sheet 141A is too large, transmission of the pulse wave signal in the blood vessel 201 to the pressure sensor 111 is affected; or if a thickness of the flexible sheet 141A is too small, protection effect of the flexible sheet 141A on the pressure sensor 111 is affected. Therefore, in some embodiments, the thickness of the flexible sheet 141A may be 1 mm to 3 mm, to balance force transmission effect and protection effect of the flexible sheet 141A.

In this embodiment of this application, the disposed flexible packaging layer 14A is configured to be attached to the surface of the wrist 200 of the user, to reduce discomfort of the user wearing the wearable device 100. In addition, the flexible packaging layer 14A is conformal with the surface of the wrist 200 of the user, so that pressure transmitted from the flexible sheet 141A to the measurement surface 1111 of the pressure sensor 111 can be basically perpendicular to the normal direction of the surface of the wrist 200 of the user, thereby improving blood pressure detection precision.

In conclusion, this embodiment of this application provides the pressure detection apparatus 10A. The flexible support layer 12A and the flexible packaging layer 14A form a flexible packaging structure of the pressure sensor. Therefore, in a pressure detection process, the pressure detection apparatus 10A can be conformal with a measured surface (for example, the surface of the wrist 100 of the user), to improve pressure detection precision.

In addition, the pressure detection apparatus 10A further includes a rigid sheet 131A configured to restrict bending deformation of the pressure sensor 111, to facilitate vertical transmission of the pressure in the pressure sensor, and further improve pressure detection precision.

It may be understood that this embodiment illustrates an example structure of the pressure detection apparatus, and a person skilled in the art may make other variations. For example, in some embodiments, the pressure detection apparatus 10A may not include the flexible packaging layer 14A.

The following describes Embodiment 2 of the pressure detection apparatus provided in this application. This embodiment may be based on Embodiment 1. Based on Embodiment 1, in this embodiment, pressure sensors located in adjacent rows at a pressure sensor layer are staggered, to increase an arrangement density of pressure sensors, thereby increasing resolution of the pressure detection apparatus.

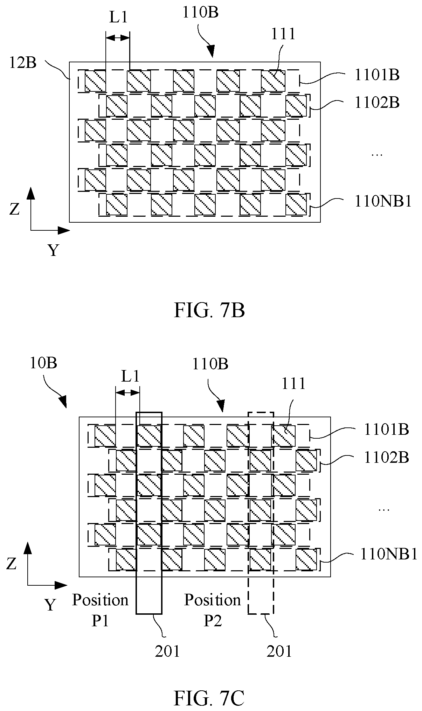

FIG. 7A and FIG. 7B show an example structure of a pressure detection apparatus 10B according to Embodiment 2 of this application. FIG. 7A is a three-dimensional diagram of a pressure detection apparatus 10B. FIG. 7B is a top view of a pressure sensor layer 11B.

As shown in FIG. 7A, the pressure detection apparatus 10B includes a flexible packaging layer 14B, a pressure sensor layer 11B, a flexible support layer 12B, and a reinforcement layer 13B that are sequentially stacked along a thickness direction.

As shown in FIG. 7A and FIG. 7B, the pressure sensor layer 11B may include a plurality of pressure sensors 111. The plurality of pressure sensors 111 may be arranged in a plurality of rows and a plurality of columns, thereby forming a sensor array 110B. A Y-axis direction is a row direction of the sensor array 110B, and a Z-axis direction is a column direction of the sensor array 110B. As shown in FIG. 7A and FIG. 7B, the sensor array 110B may include a plurality of rows, for example, a first row of sensors 1011B, a second row of sensors 1002B, ..., and an N^{th} row of sensors 110NB. Herein, N is a positive integer greater than 2. Each row may include a plurality of pressure sensors 111.

In some embodiments, a plurality of pressure sensors 111 disposed in each row of sensors are substantially the same as a plurality of pressure sensors 111 disposed in the row direction in the sensor array 110B in Embodiment 1. For details, refer to related descriptions in Embodiment 1. Details are not described again. For example, to avoid interference between adjacent sensors in a same row when a wearable device is worn on a wrist of a user, there may be a spacing L1 between adjacent sensors in a same row.

Embodiment 2 differs from Embodiment 1 of this application in that pressure sensors 111 in two adjacent rows are staggered along a width direction (namely, the Z-axis direction). In other words, a projection of a pressure sensor 111 in each row along the width direction does not overlap a projection of a pressure sensor 111 in an adjacent row along the width direction. For example, as shown in FIG. 7B, there is a gap L1 between adjacent sensors 111 in each row of sensors, and an entity part of the pressure sensor 111 in each row is aligned with a gap L1 between pressure sensors 111 in an adjacent row along the width direction. In other words, a projection of the entity part of the pressure sensor 111 in each row along the Z-axis direction at least partially overlaps a projection of the gap L1 between pressure sensors 111 in the adjacent row along the Z-axis direction.

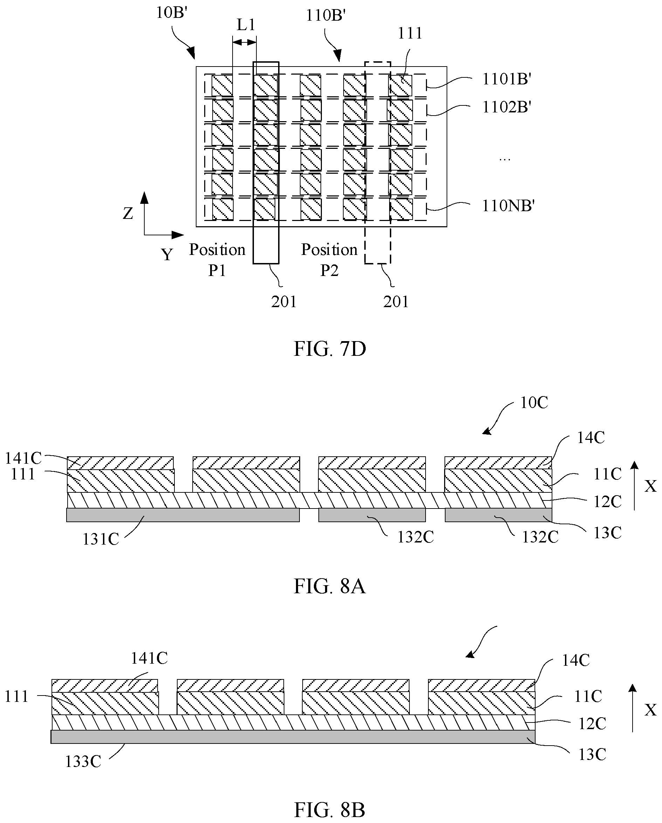

With reference to FIG. 7C and FIG. 7D, the following describes a function of staggering pressure sensors 111 in adjacent rows. FIG. 7C is a diagram of a position relationship between a sensor array 110B and a wrist blood vessel 201 of a user in this embodiment when a wearable device is worn on a wrist of the user. FIG. 7D is a diagram of a position relationship between a sensor array 110B' and a wrist blood vessel 201 in a comparative embodiment. In the comparative embodiment, pressure sensors 111 in adjacent rows are arranged in alignment.

It may be understood that when the wearable device is worn on the wrist of the user, the position relationship between the blood vessel 201 and the pressure sensor array 110B is not specified. For example, as shown in FIG. 7C and FIG. 7D, when the wearable device is worn on the wrist of the user, relative to the pressure sensor array 110B, the blood vessel 201 may be located at a position P1, or may be located at a position P2.

As shown in FIG. 7D, because there is a gap L1 between pressure sensors 111 in a same row, when the blood vessel 201 is located at the position P1, the blood vessel 201 is aligned with a column of pressure sensors 111. In this case, the pressure detection apparatus 10B' may detect blood pressure of the user by using the column of pressure sensors 111. However, when the blood vessel 201 is located at the position P2, the blood vessel 201 may be aligned with the gap between pressure sensors 111, and no pressure sensor 111 is aligned with the blood vessel 201 or a small quantity of pressure sensors 111 are aligned with the blood vessel 201. Consequently, measurement precision of the pressure detection apparatus 10B' is affected.

However, in this embodiment of this application, when the blood vessel 201 is located at the position P1, the blood vessel 201 may be aligned with a first row of sensors 1011B, a third row of pressure sensors, a fifth row of pressure sensors, and the like. When the blood vessel 201 is located at the position P2, the blood vessel 201 may be aligned with a second row of sensors 1012B, a fourth row of pressure sensors, a sixth row of pressure sensors, and the like. In other words, pressure sensors 111 in adjacent rows are staggered, to increase an arrangement density of pressure sensors 111 in the row direction, thereby more easily aligning the blood vessel 201 and the pressure sensors 111, and reducing a requirement on alignment precision between the pressure detection apparatus and the blood vessel.

In some examples, the projection of the entity part of the pressure sensor 111 in each row along the Z-axis direction may cover the projection of the gap L1 between pressure sensors 111 in the adjacent row along the Z-axis direction, to further increase an arrangement density of the pressure sensor 111 in the row direction, and reduce a requirement on alignment precision between the pressure detection apparatus and the blood vessel.

Other details that are not described in this embodiment, for example, structures of the flexible packaging layer 14B, the flexible support layer 12B, and the reinforcement layer 13B may be substantially the same as structures of the flexible packaging layer 14A, the flexible support layer 12A, and the reinforcement layer 13A in Embodiment 1. Therefore, for details, refer to the descriptions in Embodiment 1. Details are not described again.

In conclusion, in this embodiment, pressure sensors 111 in adjacent sensors are staggered, to increase an arrangement density of the pressure sensors 111 in the row direction, thereby increasing resolution of the pressure detection apparatus 10B in the row direction, and reducing a requirement on alignment precision between the pressure detection apparatus and the blood vessel.

The following describes Embodiment 3 of the pressure detection apparatus provided in this application. This embodiment may be based on Embodiment 1 and Embodiment 2. Based on Embodiment 1 and Embodiment 2, in this embodiment, the rigid sheet in the reinforcement layer is set to be in a non-one-to-one correspondence form with the pressure sensor. In other words, at least one rigid sheet corresponds to a plurality of pressure sensors. A quantity of rigid sheets in the reinforcement layer is less than a quantity of rigid sheets in the reinforcement layer in Embodiment 1 and Embodiment 2. In this way, processing difficulty and processing costs can be reduced while support and reinforcement functions for the pressure sensor are implemented.

FIG. 8A and FIG. 8B show an example structure of a pressure detection apparatus 10C according to Embodiment 3 of this application. FIG. 8A shows an example structure 1 of a pressure detection apparatus 10C according to this embodiment. FIG. 8B shows an example structure 2 of a pressure detection apparatus 10C according to this embodiment.

As shown in FIG. 8A and FIG. 8B, the pressure detection apparatus 10C includes a flexible packaging layer 14C, a pressure sensor layer 11C, a flexible support layer 12C, and a reinforcement layer 13C that are sequentially stacked along the thickness direction.

This embodiment differs from Embodiment 1 and Embodiment 2 in that the reinforcement layer 13C may include at least one rigid sheet, and the rigid sheet is not in one-to-one correspondence with the pressure sensor. In other words, each rigid sheet may correspond to one pressure sensor, or may correspond to a plurality of pressure sensors. For example, each rigid sheet may correspond to a part of sensors in the pressure sensor layer 11C.

For example, as shown in FIG. 8A, the reinforcement layer 13C may include one rigid sheet 131C and a plurality of rigid sheets 132C. The rigid sheet 131C corresponds to two pressure sensors 111. To be specific, a projection of the rigid sheet 131C along the thickness direction covers projections of the two pressure sensors 111 along the thickness direction. In this way, the rigid sheet 131C is configured to support and reinforce both the two pressure sensors 111. The rigid sheet 132C corresponds to one pressure sensor 111. To be specific, a projection of the rigid sheet 132C along the thickness direction covers a projection of the one pressure sensor 111 along the thickness direction. In other words, the rigid sheet 132C is configured to support and reinforce the one pressure sensor 111.

In some embodiments, a plurality of rigid sheets 131C may be disposed, and/or one rigid sheet 132C may be disposed. In another embodiment, the projection of the rigid sheet 131C along the thickness direction may cover projections of more than two (for example, three or five) pressure sensors 111 along the thickness direction. This is not specifically limited.

In some embodiments, as shown in FIG. 8B, the reinforcement layer 13C may include only one rigid sheet 133C. The projection of the rigid sheet 133C along the thickness direction covers projections of all pressure sensors 111 along the thickness direction. In other words, the rigid sheet 133C is configured to support and reinforce all the pressure sensors 111, thereby further reducing processing difficulty.

In some embodiments, a specific implementation in which the rigid sheet 131C, the rigid sheet 132C, and the rigid sheet 133C correspond to one or more pressure sensors for reinforcement and support is substantially the same as a specific implementation in which the rigid sheet is in one-to-one correspondence with the pressure sensor for reinforcement and support in Embodiment 1. For details, refer to related descriptions in Embodiment 1 and Embodiment 2. Details are not described again.

It may be understood that the flexible packaging layer 14C, the pressure sensor layer 11C, and the flexible support layer 12C in the pressure detection apparatus 10C are substantially the same as the flexible packaging layer, the pressure sensor layer, and the flexible support layer in Embodiment 1 and Embodiment 2. For details, refer to related descriptions in Embodiment 1 and Embodiment 2. Details are not described again.

In conclusion, in this embodiment, at least one rigid sheet is disposed in the reinforcement layer, and the rigid sheet and the pressure sensor are arranged in a non-one-to-one correspondence form, to reduce a quantity of rigid sheets, and reduce processing difficulty and processing costs while implementing support and reinforcement functions for the pressure sensor.

The following describes Embodiment 4 of the pressure detection apparatus provided in this application. This embodiment may be based on the foregoing three embodiments. Based on Embodiment 1, Embodiment 2, and Embodiment 3, in this embodiment, the flexible sheet in the flexible packaging layer is set to be in a non-one-to-one correspondence form with the pressure sensor. In other words, at least one flexible sheet corresponds to a plurality of pressure sensors. A quantity of flexible sheets in the flexible packaging layer is less than a quantity of flexible sheets in the flexible packaging layer in the foregoing three embodiments, to reduce processing difficulty and costs.

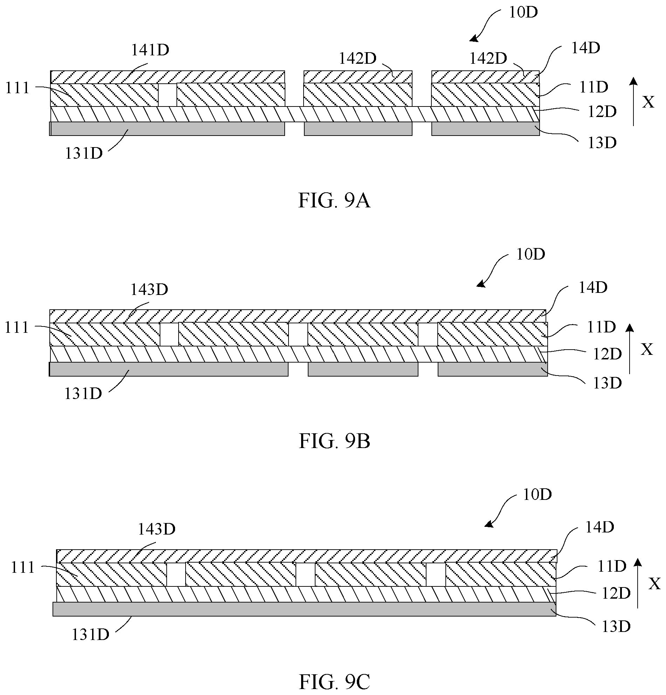

FIG. 9A to FIG. 9C show an example structure of a pressure detection apparatus 10D according to Embodiment 4 of this application. FIG. 9A shows a front view of a first pressure detection apparatus 10D. FIG. 9B shows a front view of a second pressure detection apparatus 10D. FIG. 9C shows a front view of a third pressure detection apparatus 10D.

As shown in FIG. 9A and FIG. 9B, the pressure detection apparatus 10D includes a flexible packaging layer 14D, a pressure sensor layer 11D, a flexible support layer 12D, and a reinforcement layer 13D that are sequentially stacked along the thickness direction.

Some embodiments differ from the foregoing three embodiments in that the flexible packaging layer 14D may include at least one flexible sheet, and the flexible sheet is not in one-to-one correspondence with the pressure sensor. For example, as shown in FIG. 9A, the flexible packaging layer 14D may include one flexible sheet 141D and a plurality of flexible sheets 142D. A projection of the flexible sheet 141D along the thickness direction covers projections of two pressure sensors 111 along the thickness direction. A projection of the flexible sheet 142D along the thickness direction covers a projection of one pressure sensor 111 along the thickness direction.

In some embodiments, a plurality of flexible sheets 141D may be disposed, and/or one flexible sheet 142D may be disposed. In another embodiment, the projection of the flexible sheet 141D along the thickness direction may cover projections of more than two (for example, three or four) pressure sensors 111 along the thickness direction. This is not specifically limited.

In some embodiments, as shown in FIG. 9B, the flexible packaging layer 14D may include only one flexible sheet 143D, and a projection of the flexible sheet 143D along the thickness direction covers projections of all pressure sensors 111 along the thickness direction.

It may be understood that the reinforcement layer 13D, the pressure sensor layer 11D, and the flexible support layer 12D in the pressure detection apparatus 10D are substantially the same as the flexible packaging layer, the pressure sensor layer, and the flexible support layer in Embodiment 1, Embodiment 2, and Embodiment 3. For details, refer to related descriptions in Embodiment 1, Embodiment 2, and Embodiment 3. Details are not described again.

For example, as shown in FIG. 9C, based on Embodiment 3, in this embodiment, the flexible sheet 143D in the flexible packaging layer 14D corresponds to all the pressure sensors 111, and the rigid sheet 141D in the reinforcement layer 13D corresponds to all the pressure sensors 111, to further reduce processing difficulty and processing costs.

In conclusion, in this embodiment, at least one flexible sheet is disposed at the flexible packaging layer, and the flexible sheet and the pressure sensor are arranged in a non-one-to-one correspondence form, to reduce a quantity of flexible sheets, and reduce processing difficulty and processing costs.

It should be noted that, orientation terms such as "up", "down", "left", "right", "front", "back", "top", and "bottom" in this specification are example orientations shown in the accompanying drawings, but do not indicate or imply that a specified component needs to have a specific orientation. The orientation terms may change correspondingly based on actual use, and cannot be construed as a limitation on this application.

In the foregoing descriptions of this embodiment, unless otherwise specified, "/" means "or". For example, A/B may identify A or B. In this specification, "and/or" describes only an association relationship between associated objects and represents that three relationships may exist. For example, A and/or B may represent the following three cases: Only A exists, only B exists, and both A and B exist.

## Claims

1. A pressure detection apparatus, comprising:
a plurality of pressure sensors;
a flexible support layer, wherein the flexible support layer comprises a first surface and a second surface that are disposed back to back along a first direction, the plurality of pressure sensors are spaced apart and attached to the first surface of the flexible support layer, and the plurality of pressure sensors are mechanically connected by using at least the flexible support layer; and
one or more rigid sheets, wherein the one or more rigid sheets are attached to the second surface of the flexible support layer, wherein
each of the one or more rigid sheets corresponds to a part of the plurality of pressure sensors, and a projection of each rigid sheet along the first direction covers a projection of the pressure sensor corresponding to the rigid sheet along the first direction.

2. The pressure detection apparatus according to claim 1, wherein there are a plurality of rigid sheets, and the plurality of rigid sheets are in one-to-one correspondence with the plurality of pressure sensors.

3. The pressure detection apparatus according to claim 1 or 2, wherein there are a plurality of rigid sheets, and the plurality of rigid sheets are spaced apart from each other.

4. The pressure detection apparatus according to any one of claims 1 to 3, wherein the pressure detection apparatus further comprises a flexible packaging layer, and the flexible packaging layer is disposed on a side that is of the pressure sensor layer and that faces away from the flexible support layer; and
a projection of the flexible packaging layer along the first direction covers at least a projection of a part of the plurality of pressure sensors along the first direction.

5. The pressure detection apparatus according to claim 4, wherein the flexible packaging layer comprises one or more flexible sheets spaced apart, each of the one or more flexible sheets corresponds to a part of the plurality of pressure sensors, and a projection of each flexible sheet along the first direction covers a projection of the pressure sensor corresponding to the flexible sheet along the first direction.

6. The pressure detection apparatus according to claim 5, wherein there are a plurality of flexible sheets, and the plurality of flexible sheets are in one-to-one correspondence with the plurality of pressure sensors.

7. The pressure detection apparatus according to claim 5 or 6, wherein the flexible sheet is attached to the pressure sensor corresponding to the flexible sheet.

8. The pressure detection apparatus according to any one of claims 1 to 7, wherein the flexible support layer comprises a flexible substrate and a conducting wire disposed on the flexible substrate, and the plurality of pressure sensors are mechanically connected by using the flexible substrate and electrically connected by using the conducting wire.

9. The pressure detection apparatus according to any one of claims 1 to 8, wherein the plurality of pressure sensors are arranged in one or more rows, and each row comprises a plurality of the pressure sensors.

10. The pressure detection apparatus according to claim 9, wherein the plurality of pressure sensors are arranged in a plurality of rows, the pressure sensors in a same row are spaced apart along a second direction, and the second direction is perpendicular to the first direction; and
the pressure sensors in two adjacent rows are staggered along a third direction, and the third direction is perpendicular to the second direction and is perpendicular to the first direction.

11. The pressure detection apparatus according to claim 9, wherein a gap between adjacent pressure sensors in a same row is 0.1 mm to 1 mm.

12. The pressure detection apparatus according to any one of claims 1 to 11, wherein a dimension of the pressure sensor along the first direction is less than or equal to 0.5 mm; and/or
a dimension of the pressure sensor along the second direction is less than or equal to 1 mm, and the second direction is perpendicular to the first direction; and/or
a dimension of the pressure sensor along the third direction is less than or equal to 1 mm, and the third direction is perpendicular to the second direction and is perpendicular to the first direction.

13. The pressure detection apparatus according to any one of claims 1 to 11, wherein a material of the flexible substrate layer comprises at least one of polydimethylsiloxane, an elastomer compound, a flexible resin, and a flexible silicone.

14. The pressure detection apparatus according to any one of claims 1 to 13, wherein the pressure sensor is an MEMS pressure sensor.

15. The pressure detection apparatus according to claim 14, wherein the MEMS pressure sensor is a silicon-based piezoresistive sensor, a silicon-based piezoelectric sensor, or a silicon-based capacitive sensor.

16. An electronic device, comprising a main body and the pressure detection apparatus according to any one of claims 1 to 15, wherein the pressure detection apparatus is disposed on a surface of the main body.

17. The electronic device according to claim 16, wherein the electronic device is a wearable device.

18. The electronic device according to claim 17, wherein the wearable device is a wristband-type device.
